# EUROPEAN PATENT SPECIFICATION

(11) **EP 2 643 396 B1**
(45) Date of publication and mention of the grant of the patent: **13.03.2019**
(21) Application number: 11842961.2
(22) Date of filing: 24.11.2011
(51) Int. Cl.: A61K 9/51, A61K 9/50, A61L 27/18, C08J 7/06, C08J 3/12, B82Y 30/00, A61L 27/32

(54) **METHOD FOR COATING PARTICLES WITH CALCIUM PHOSPHATE AND PARTICLES, MICROPARTICLES AND NANOPARTICLES FORMED THEREOF**
VERFAHREN ZUR BESCHICHTUNG VON PARTIKELN MIT CALCIUMPHOSPHAT SOWIE DARAUS GEFORMTE PARTIKEL, MIKROPARTIKEL UND NANOPARTIKEL
PROCÉDÉ DE REVÊTEMENT DE PARTICULES AVEC DU PHOSPHATE DE CALCIUM ET PARTICULES, MICROPARTICULES ET NANOPARTICULES FORMÉES AVEC CELUI-CI

(30) Priority: 24.11.2010 US 416973 P
(43) Date of publication of application: 02.10.2013
(73) Proprietor: Nanyang Technological University, Singapore 639798 (SG)
(72) Inventor: LOO, Say Chye Joachim, Singapore 637722 (SG); BASTARI, Kelsen, Singapore 637722 (SG); VENKATRAMAN, Subramaniam, Singapore 637722 (SG)
(74) Representative: Viering, Jentschura & Partner mbB Patent- und Rechtsanwälte
(86) International application number: PCT/SG2011/000414
(87) International publication number: WO 2012/071014

(56) References cited:
- WO-A1-00/00177
- WO-A1-2010/036919
- WO-A2-2008/149096
- WO-A2-2008/149096
- US-A- 6 153 266
- US-A- 6 153 266

## Description

### Technical Field

The invention relates to a method of coating particles, and in particular, to a method of coating particles with calcium phosphate. Nanoparticles formed thereof are also provided.

### Background

Calcium phosphate (CaP) is a family of minerals that contains calcium cations and phosphate anions, of which these could be orthophosphates, metaphosphates or pyrophosphates. These compounds are of great interest in an interdisciplinary field of sciences involving chemistry, biology, and medicine. In recent years, CaP has been receiving considerable attention in the biomedical sector, especially in orthopedics, because it is biocompatible, biodegradable, and is the main mineral component of bones. Also, the use of CaP in biomedical applications has shown to improve bone bonding, cell adhesion, and new bone formation.

Particulate systems, i.e. microparticles and nanoparticles, have been utilized to deliver therapeutic agents to targeted tissues. Besides targeted delivery, these particles also protect drugs from degradation until they reach the targeted site. Bone-targeting, through the use of particles, therefore opens a wide platform for treatment of various bone diseases, i.e. osteomyelitis, osteosarcoma etc. Current strategies in bone drug delivery involve the use of biodegradable polymeric particles to bones. However, these strategies have several drawbacks, including poor bone targeting capabilities, formation of acidic degradation by-products, and a lack of bioactivity. An ideal bone-targeting system should therefore have good targeting capabilities, and properties close to bone tissues to promote bone formation and encourage healing.

WO2008/149096 discloses microparticles coated with calcium phosphate. Negatively charged PLGA microparticles are contacted with a supersaturated solution of calcium phosphate.

### Summary

According to a first aspect of the invention, there is provided a method for coating particles which are negatively charged with calcium phosphate (CaP). The method is defined in the claims.

According to a further aspect of the invention, there is provided a nanoparticle comprising a negatively charged particle coated with calcium phosphate (CaP), wherein calcium ions of the CaP are absorbed onto the surface of the negatively charged particle, and wherein the nanoparticle has a diameter of 50 to 500nm. The nanoparticle may be useful for drug delivery.

### Brief Description of the Drawings

In the drawings, like reference characters generally refer to the same parts throughout the different views. The drawings are not necessarily drawn to scale, emphasis instead generally being placed upon illustrating the principles of various embodiments. In the following description, various embodiments of the invention are described with reference to the following drawings.
**Figure 1** illustrates the present method of coating a particle with CaP.
**Figure 2** shows SEM images of PLGA microspheres before (a) and after (b) CaP coating, the microspheres having diameter in the range of 100-150µm.
**Figure 3** shows FESEM images of the present CaP-coated PLGA nanoparticles..
**Figure 4** shows SEM images of (a) bare PLGA particles and (b) the present CaP-coated PLGA particles having diameter in the range of 300-400nm.
**Figure 5** shows SEM images of (a) bare PLGA particles and (b) the present CaP-coated PLGA particles having diameter in the range of 2-15µm.
**Figure 6** shows SEM images of the present CaP-coated PLGA particles obtained by using 2.5mM Ca solution and 1.5mM P solution at (a) 10,000x magnification and (b) 30,000x magnification, stirred for 10 and 2.5 minutes for Ca and P solutions, respectively.
**Figure 7** shows SEM images of the present CaP-coated PLGA particles obtained by using 1.25mM Ca solution and 0.75mM P solution at (a) 15,000x magnification and (b) 30,000x magnification, stirred for 10 and 2.5 minutes for Ca and P solutions, respectively.
**Figure 8** shows SEM images of the present CaP-coated PLGA particles obtained by using 0.625mM Ca solution and 0.375mM P solution at (a) 15,000x magnification and (b) 30,000x magnification, stirred for 10 and 2.5 minutes for Ca and P solutions, respectively.
**Figure 9** shows the drug nafcillin release profile for bare PLGA and CaP-coated PLGA particles.

### Description

There is provided a method for coating particles with calcium phosphate (CaP). The CaP-coated particles may be ceramic-polymer hybrid particles and are suitable for, but not limited to, orthopedic applications. These particles can serve as a drug delivery carrier and yet at the same time have surface properties similar to that of bone tissues.

The present method is a simple, reliable, economical and versatile technique to produce particles coated with CaP. The synthesis of the CaP-coated particles is conducted through a surface reaction that allows CaP to coat onto the particles. Advantageously, this technique also allows for a uniform coating of CaP onto the particles with close to 100% efficiency. Drug-loaded particles can also be synthesized.

In the present context, as mentioned earlier, CaP is a family of minerals that contains calcium cations and phosphate anions. Non-limiting examples of such minerals can for example include, hydroxyapatite (Ca₁₀(PO₄)₆(OH)₂) or suitable precursors thereof; dicalcium phosphate; tricalcium phosphate; *β*-tricalcium phosphate (*β*-Ca₃PO₄); mono or biphasic calcium phosphates; composites of calcium sulphate and hydroxyapatite (PerOssal®); composites of hydroxyapatite; calcium deficient apatite (Ca₁₀₋ₓ(PO₄)₆₋ₓ(HPO₄)ₓ(OH)₂₋ₓ) or combinations thereof.

In various embodiments where the particles serve as a drug carrier, the carrier can be customized into any designs or shapes so that the drug for example, can be incorporated thereon or therein. The carrier can, for example, be in the form of a core-shell structure whereby the drug is encapsulated within the core and is released upon reaching the targeted site.

Any drug or medicament can be incorporated into the particles of the invention as long as the desired purpose is achieved. The term "drug" as used herein refers to any therapeutically active agent or pharmaceutically active agent and is generally accepted in the art to be any compound or substance which is used to treat or prevent any given disease or disorder or in the regulation of a physiological condition in a human or animal subject. In some embodiments, the drug can be an antibiotic, an antifungal, a peptide, a protein, a polymer, a nucleic acid molecule, or combinations thereof. Exemplary antibiotics can include but are not limited to vancomycin, gentamicin, amoxicillin, imipenem, amphotericin B, cefoperazone, doxycycline and combinations thereof, to mention only a few.

In the present context, the particles to be coated may comprise a polymer or polymer mixture. Any polymeric material that is within the knowledge of the average skilled person can be used for this purpose. Such polymeric material can be of linear or branched polymers, homopolymers, blockpolymers, copolymers, or mixtures thereof. Examples of such polymeric material can include but are not limited to poly(lactide-co-glycolic acid) (PLGA), polymethylacrylate (PMMA), polyethylene glycol (PEG), poly(propylene glycol-fumerate), polylactic acid (PLA), poly(L-lactic) (PLLA), polycaprolactone (PCL) or combinations thereof. The particles comprising the polymer or polymer mixture are negatively charged, either as an intrinsic polymer property or through the use of surface agents, such as surfactants.

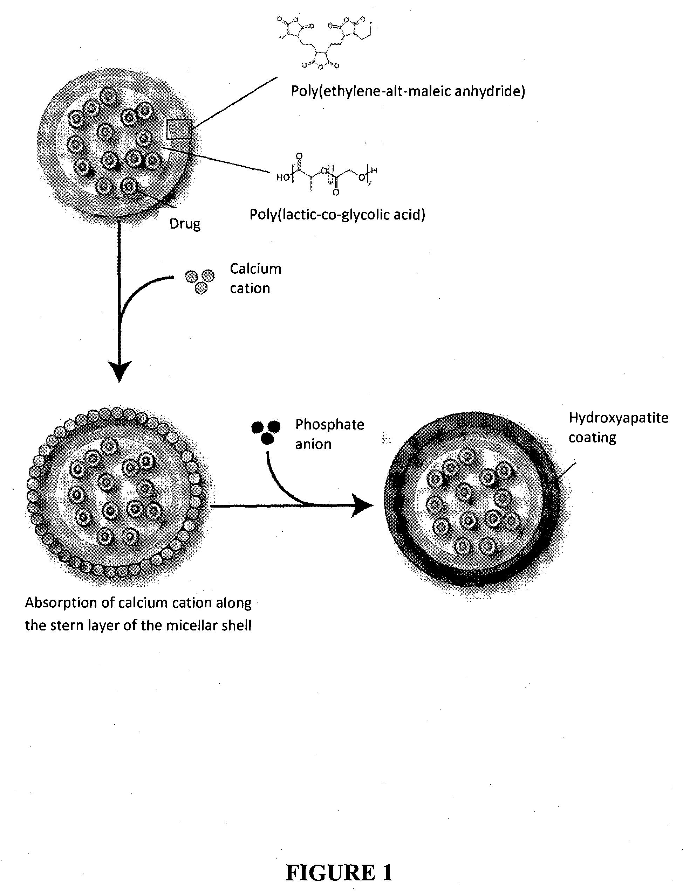

**Figure 1** illustrates an embodiment of the present method. The method includes contacting the particles with a first solution containing calcium ions, wherein the particles are negatively charged. In the embodiment as shown, the particles are PLGA and are formed of a surface-modified structure with the use of surfactants. A plurality of drug compounds are encapsulated within the core of the PLGA particle.

Any solution containing calcium ions in adequate concentration may be used as the first solution in the present method. In various embodiments, the first solution may be selected from the group consisting of calcium nitrate tetrahydrate solution and calcium chloride. In one embodiment, the first solution is calcium nitrate tetrahydrate solution formed by dissolving calcium nitrate tetrahydrate (Ca(NO₃)₂.4H₂O) in distilled water. A base such as ammonium hydroxide may be further added to the first solution to maintain the pH in the range of about 10 to 12. pH affects the type of calcium phosphate formed. A basic pH would be preferred to achieve the formation of hydroxyapatite, the calcium phosphate compound naturally present in bones.

The particles may be contacted with the first solution by adding the particles to the first solution and stirring the solution for between about 5 minutes and about 5 hours. In various embodiments, the solution may be stirred for between about 10 minutes and 3 hours. The stirring may be carried out by a magnetic stirrer, mechanical stirrer, shaker, or any other common stirring method.

PLGA particles are excellent drug carriers and they allow for controlled drug release. However, degradation of PLGA leaves behind acidic by-products which are undesirable for bone applications. The presence of CaP will help buffer the degradation environment and promote bone healing. CaP coating can also provide an anchoring site for attachment of specific ligands required in targeting, i.e. bisphosphonate for bone targeting. In addition, owing to its similar composition to natural bone tissue, CaP coating can promote cell adhesion, bone bonding and formation and accelerate the healing process. Coating CaP onto PLGA particles is therefore desirable for bone-related applications or applications in close proximity to bones.

After allowing the absorption of calcium ions onto the particles, the first solution is removed to collect the particles in a form of a precipitate and the precipitate is then contacted with a second solution containing phosphate ions to obtain the CaP-coated particles.

The first solution may be removed by any conventional removal technique such as filtration or centrifugation.

The solution is selected from the group consisting of ammonium dihydrogenphosphate solution, disodium hydrogen phosphate, dipotassium hydrogen phosphate, and orthophosphoric acid. In one embodiment, the second solution is ammonium dihydrogenphosphate solution formed by dissolving ammonium dihydrogenphosphate (NH₄H₂PO₄) in distilled water. A base such as ammonium hydroxide may be further added to the second solution to maintain the pH in the range of about 10 to 12. A basic pH would be preferred to achieve the formation of hydroxyapatite, the calcium phosphate compound naturally present in bones.

The precipitate may be contacted with the second solution containing phosphate ions by adding the precipitate to the second solution and stirring the solution for between about 2 minutes and 3 days. In various embodiments, the solution may be stirred for between about 2.5 minutes and 2 days. The stirring may be carried out by a magnetic stirrer, mechanical stirrer, shaker, or any other common stirring method.

In a further optional step, the CaP-coated particles may be collected by removing the second solution. For removal of the second solution, similar techniques as those employed for removing the first solution and described above can be used.

By adding the positively charged calcium ions to the negatively charged PLGA particles, a layer of calcium ions attach firmly to the negatively charged PLGA particles surface forming a stern layer, as illustrated in **Figure 1****.** Electrostatic interactions between calcium ions and the negatively charged PLGA particles at the stern layer of negatively charged PLGA particles occur, allowing the absorption of calcium ions at the stern layer. Once the phosphate solution is added, the reaction between Ca²⁺ ions from Ca(NO₃)₂.4H₂O and (PO₄)³⁻ ions from NH₄H₂PO₄ in basic condition will form hydroxyapatite, (Ca₁₀(PO₄)₆(OH)₂ based on the following stoichiometric equation:

10Ca(NO₃)₂ + 6NH₄H₂PO₄ + 14NH₄OH → Ca₁₀(PO₄)₆(OH)₂ + 20NH₄NO₃ + 12H₂O.

The absorption of the Ca ions to the stern layer of the particles and then the hydroxyapatite formation on the surface enables the growth and propagation of CaP layer more homogenously along the surface of the PLGA particle. This helps to ensure that subsequent drug release kinetic will not be compromised by uneven surface coating.

The obtained nanoparticles have an average diameter of 50 to 500nm. Due to the intrinsic electrostatic interaction of the PLGA particle surface and the calcium ion, thickness across the surface of the present particle may be better modulated, even at the nano level regime. The thickness of CaP crystal on the particle surface is of upmost importance with regard to further modulation of surface chemistry as well as delivery/administration of drugs. The present method represents a refinement in controlling the surface thickness as well as the overall fabrication scheme, particularely for drug delivery applications where acquiring nano-sized particles is certainly desired.

In one embodiment, the particles are produced by the emulsion solvent evaporation method comprising dissolving the particle material and a suitable solvent, adding the solution to an aqueous solution containing a negatively charged surfactant to form an emulsion, adding the emulsion to an aqueous solution containing a negatively charged surfactant and evaporating the solvent; and collecting the negatively charged particles from the aqueous solution. The particles thus formed may further comprise a negatively charged compound. In various embodiments, the compound is a surfactant.The surfactant acts to stabilise the particle material during the emulsion solvent evaporation process. The surfactant may form a micellar shell layer surrounding the polymeric core of the core-shell structured particle. In various embodiments, the surfactant is selected from the group consisting of sodium dodecyl sulfate (SDS), poly(ethylene-alt-maleic anhydride) (PEMA) and poly(vinyl alcohol) (PVA).

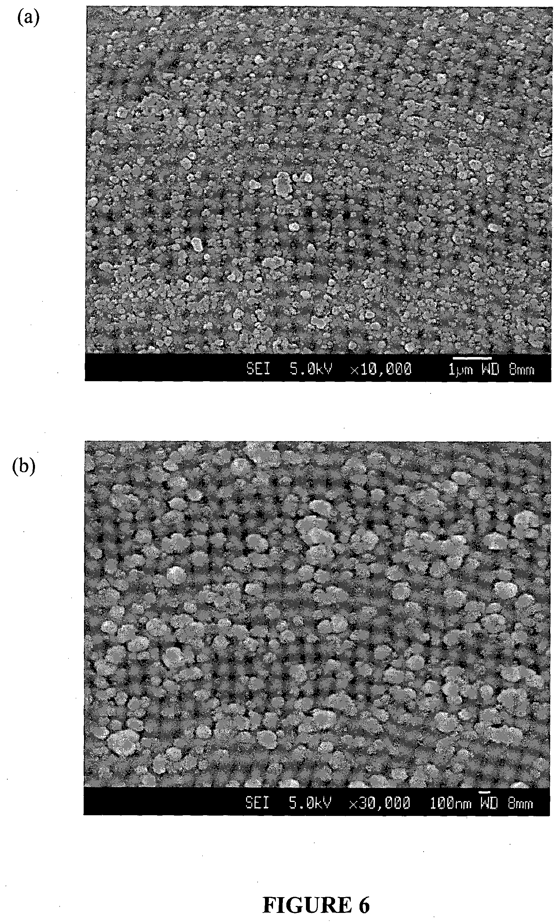

In various embodiments, the ratio of concentration of the first solution to the concentration of the second solution is in the range of about 5:1 to about 1:1, such as about 4:1, 3:1, 2:1, and 5/3:1. It has been found that by adjusting the concentration of both the first and second solutions, the time required for stirring the respectively solution could be reduced drastically to 10 and 2.5 minutes, for example, for the first and second solution, respectively. This shortened stirring timing is indeed very desirable and attractive because in addition to cutting down the synthesis period, it can also minimize the drug loss if these particles are loaded with drugs. **Figures 6****,** **7** and **8** shows that concentrations of the first (i.e. Ca) solution and the second (i.e. P) solution play a very important role to produce a uniformly coating of CaP onto PLGA particles within a shortened stirring period.

In various embodiments, the method further includes separating the CaP-coated particles from the second solution. For example, the separation may be achieved by centrifuging the mixture to obtain the CaP-coated particles. Subsequent washing and freeze-drying steps may also be carried out after obtaining the CaP-coated particles.

Any references in the description to treatments or to methods of treatment refer to the compositions or formulations of the present invention for use in a method of treatment. CaP-coated PLGA particles can be used as drug carriers in treating various diseases. Some of these include bone diseases, such as osteomyletis and osteosarcoma. For example, CaP-coated PLGA particles can be employed in treating osteomyelitis. Osteomyelitis is an acute or chronic bone infection, which may be caused by different strains of bacteria. Current treatment of osteomyelitis involves administering aggressive doses of antibiotics systemically over a period of several weeks (4-6 weeks). This current treatment strategy has many setbacks, which include inadequate antibiotic treatment of patients (because of poor drug targeting and short half-lives of drugs) and the potential for drug resistance in bacteria. In addition, because of poor treatment efficacy, acute osteomyelitis may lead to chronic osteomyelitis where the prognosis is often worse. Chronic osteomyelitis is a severe, persistent, and sometimes incapacitating infection of bone, which may result from inadequately treated acute osteomyelitis, infection with organisms and contiguous spread from soft tissues, as in diabetic ulcers. It is often a recurring condition because it is difficult to treat definitively and surgery is sometimes required for such cases. In extreme cases, amputation is needed if the infection persists and does not clear with any other treatments. All these issues may be overcome if drugs can be targeted to infected bones, through the presently disclosed delivery system employing CaP-coated PLGA particles as drug carriers.

### Examples

### Materials

Poly (lactic-co-glycolic acid) (PLGA) (53:47) with an inherent viscosity of 1.05 dL/g was purchased from Purac. Dichloromethane (DCM) and ammonia solution (NH₄OH) were supplied by Tedia and Merck respectively. Poly (vinyl alcohol) (PVA), 87-90% hydrolyzed, average molecular weight is 30,000-70,000), Poly (ethylene-alt-maleic anhydride) (PEMA), nafcillin sulfate (GS), calcium nitrate tetrahydrate (Ca(NO₃)₂.4H₂O), and ammonium dihydrogenphosphate (NH₄H₂PO₄) were all purchased from Sigma Aldrich. Phosphate buffer saline (PBS) (pH 7.4) was from Ohme Scientific.

### Fabrication of Negatively Charged PLGA Microspheres

PLGA microspheres were first prepared using emulsion solvent evaporation method with PEMA as the surfactant. Briefly, 200mg of PLGA was first dissolved in 2 ml of dichloromethane (DCM) to form an organic phase. It was then poured to 100 ml of 1% PEMA solution, which is the aqueous phase, to form oil in water emulsion. It was then emulsified using an overhead stirrer at 1000 rpm for 3 hours to allow evaporation of the organic solvent. After which, the particles were collected for CaP coating using centrifugation, washed 3 times by deionised water, frozen for 3 hours and lyophilized overnight.

### Fabrication of CaP-coated PLGA Microspheres

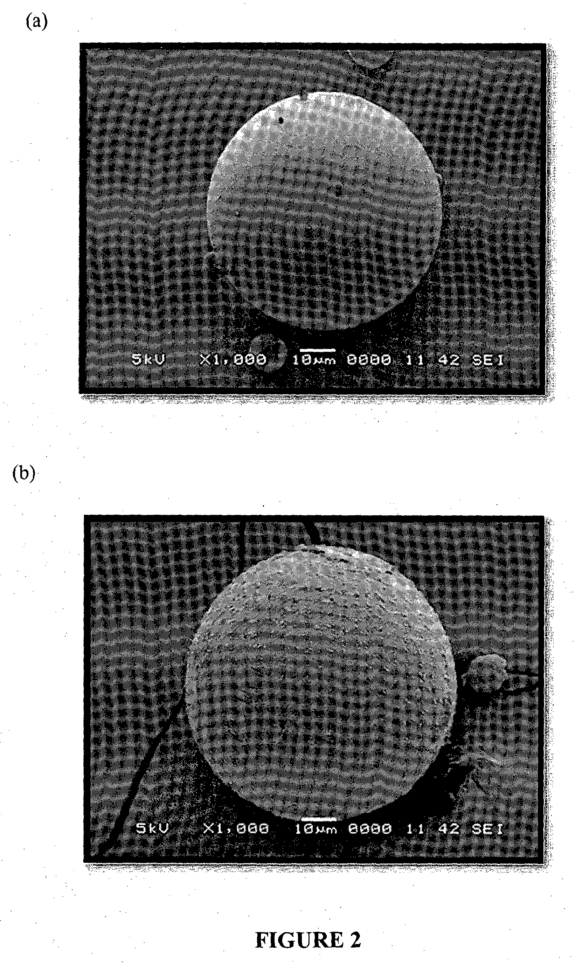

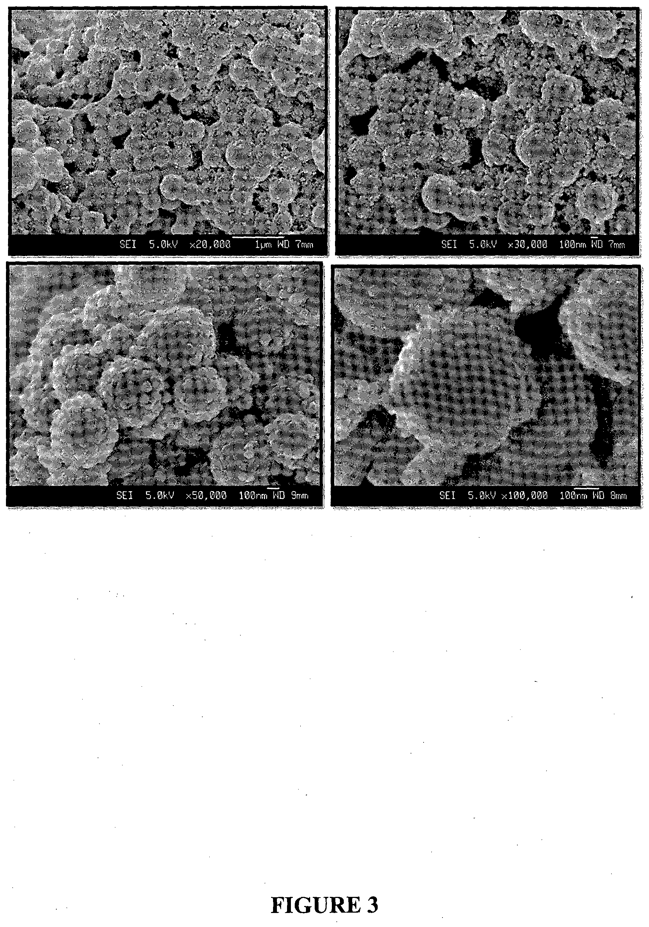

Calcium and phosphate solutions were prepared by dissolving calcium nitrate tetrahydrate (Ca(NO₃)₂.4H₂O) and ammonium dihydrogenphosphate (NH₄H₂PO₄) in distilled water. Ammonia hydroxide (NH₄OH) was added to maintain the pH of around 11. The freshly prepared negatively charged PLGA was added by 2.5 mM calcium solution and stirred for 15 minutes. After removal of the calcium solution, 1.5 mM phosphate solution was next added to the precipitate and stirred for 15 minutes. The resultant CaP-coated PLGA microspheres were collected by centrifugation, washed before freeze drying. PLGA microsphere with and without CaP coating is shown in **Figure 2****,** where the microspheres have diameters in the range of 100-150µm. **Figure 3** shows the FESEM images, at different magnifications, of the produced CaP-PLGA microspheres.

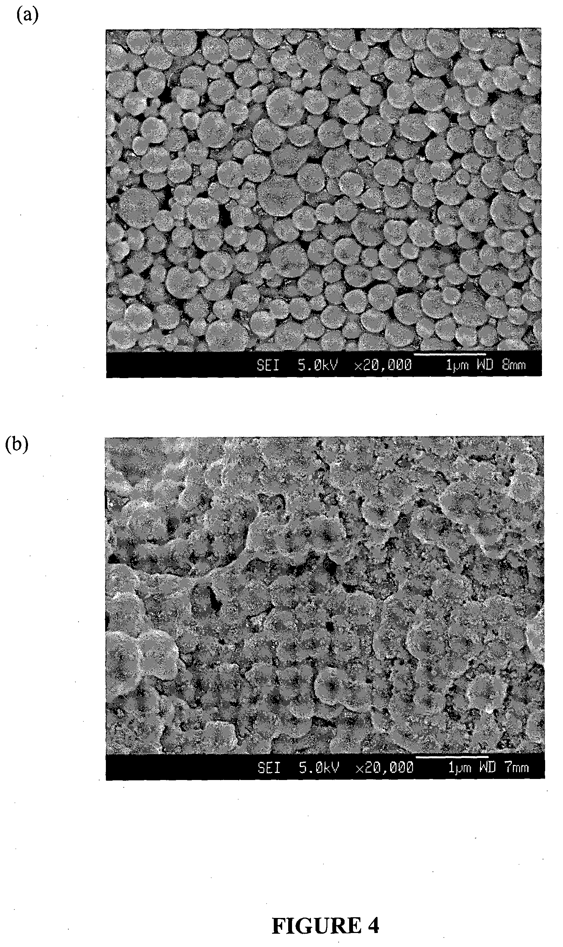

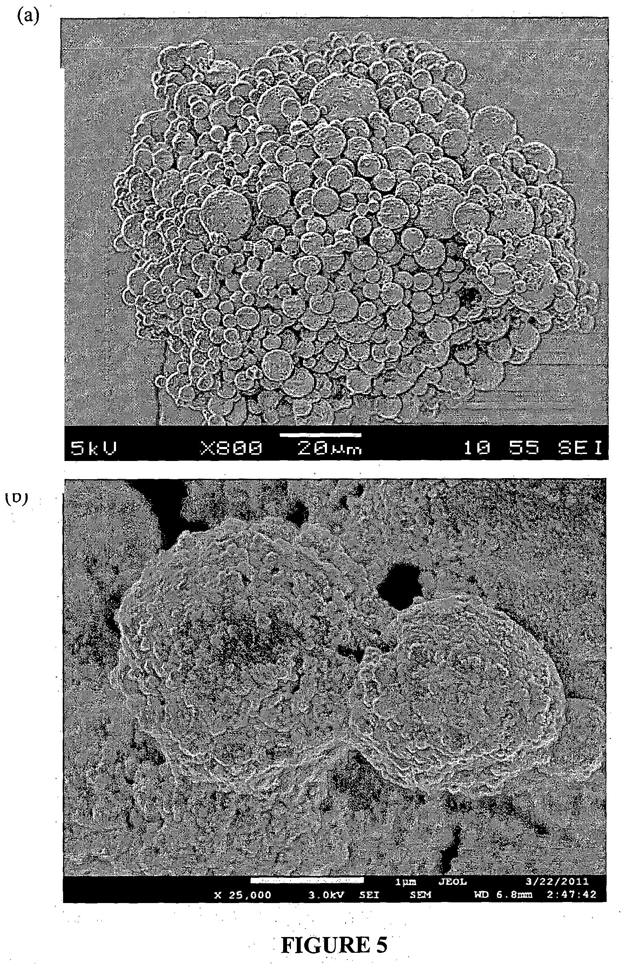

Using the same technique as described above, the coating of CaP layer onto PLGA particles had been demonstrated for different sizes of PLGA. PLGA particles at nano and sub-micron range are shown in **Figures 4** and **5****,** respectively. For sub-micron-sized range, 200mg of PLGA was first dissolved in 2 ml of dichloromethane (DCM) to form an organic phase. It was then poured to 100 ml of 1% PEMA solution to form oil in water emulsion. It was the emulsified using an overhead stirrer at 3000 rpm for 3 hours to allow evaporation of the organic solvent. After which, the particles were collected for CaP coating using centrifugation, washed 3 times by deionised water, frozen for 3 hours and lyophilized overnight. For nano-sized range, 200mg of PLGA was first dissolved in 2 ml of dichloromethane (DCM) to form an organic phase. It was then poured to 10 ml of 1% PEMA solution and was ultrasonicated using an ultrasonic probe for 2 minutes to form oil-in-water emulsion (O/W). The emulsion was subsequently poured to 90 ml of PEMA solution (1% w/v) and it was emulsified using an overhead stirrer at 1000 rpm for 3 hours to allow evaporation of the organic solvent simultaneously. After which, the particles were collected using centrifugation, washed 3 times by deionised water, frozen for 3 hours and lyophilized overnight.

The top row of **Figures 4** and **5** show PLGA particles at different size ranges without CaP coating and the one below shows the particles after CaP coating. It proved that the technique that was used could be employed to different size ranges with similar results: CaP nanoparticles were coated uniformly on the surface of the particles.

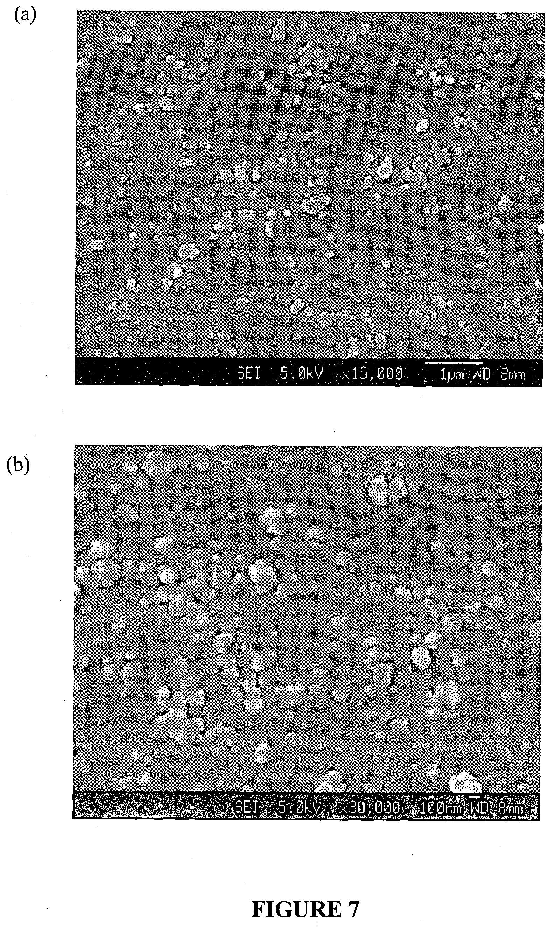

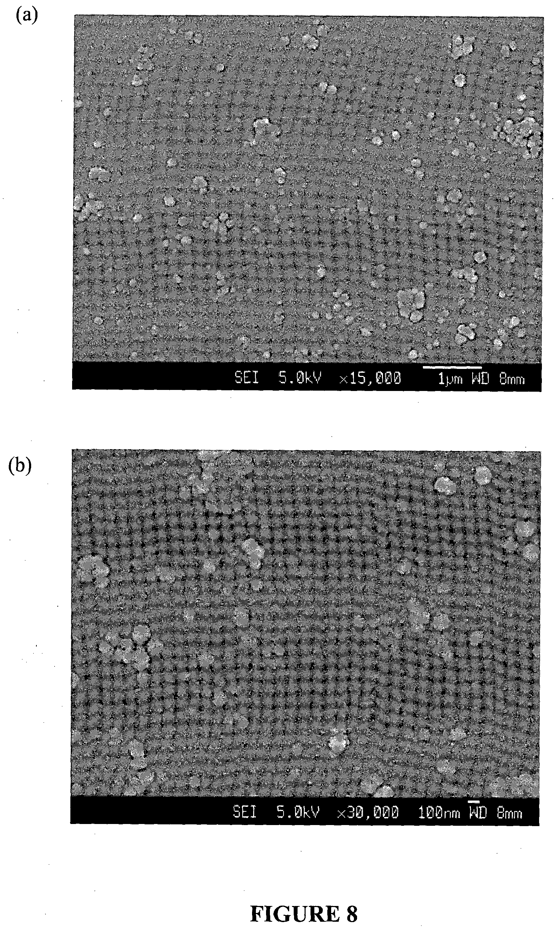

Further study also shows that reducing the stirring time during immersion in Ca and P solutions could also produce the same results as demonstrated above. By adjusting the concentration of both solutions, it was found that at the right concentrations the time required could be shortened to 10 and 2.5 minutes for Ca and P solutions, respectively. This shortened stirring timing is indeed very desirable and attractive because in addition to cutting down the synthesis period, it can also minimize the drug loss if these particles are loaded with drugs. **Figures 6****,** **7** and **8** show that concentrations of Ca and P solutions play a very important role to produce a uniformly coating of CaP onto PLGA particles within a shortened synthesis period. **Figure 6** shows SEM images of the present CaP-coated PLGA particles obtained by using 2.5mM Ca solution and 1.5mM P solution at (a) 10,000x magnification and (b) 30,000x magnification, stirred for 10 and 2.5 minutes for Ca and P solutions, respectively. **Figure 7** shows SEM images of the present CaP-coated PLGA particles obtained by using 1.25mM Ca solution and 0.75mM P solution at (a) 15,000x magnification and (b) 30,000x magnification, stirred for 10 and 2.5 minutes for Ca and P solutions, respectively. **Figure 8** shows SEM images of the present CaP-coated PLGA particles obtained by using 0.625mM Ca solution and 0.375mM P solution at (a) 15,000x magnification and (b) 30,000x magnification, stirred for 10 and 2.5 minutes for Ca and P solutions, respectively.

### Fabrication of Drug-encapsulated CaP-coated PLGA Particles

105 mg of PLGA were first dissolved in 2 ml of DCM to form the organic phase and 45 mg of GS was dissolved in 0.2 ml PVA (0.5% w/v) to form the inner aqueous phase. The drug solution was then poured to the polymer solution and ultrasonicated for 2 minutes using an ultrasonic probe to form water-in-oil emulsion (W/O). The emulsion was subsequently poured to 100 ml of PEMA solution (1% w/v) to form water-in-oil-in-water emulsion and it was emulsified using an overhead stirrer at 1000 rpm for 3 hours to allow evaporation of the organic solvent simultaneously. After which, the particles were collected using centrifugation, washed 3 times by deionised water, frozen for 3 hours and lyophilized overnight.

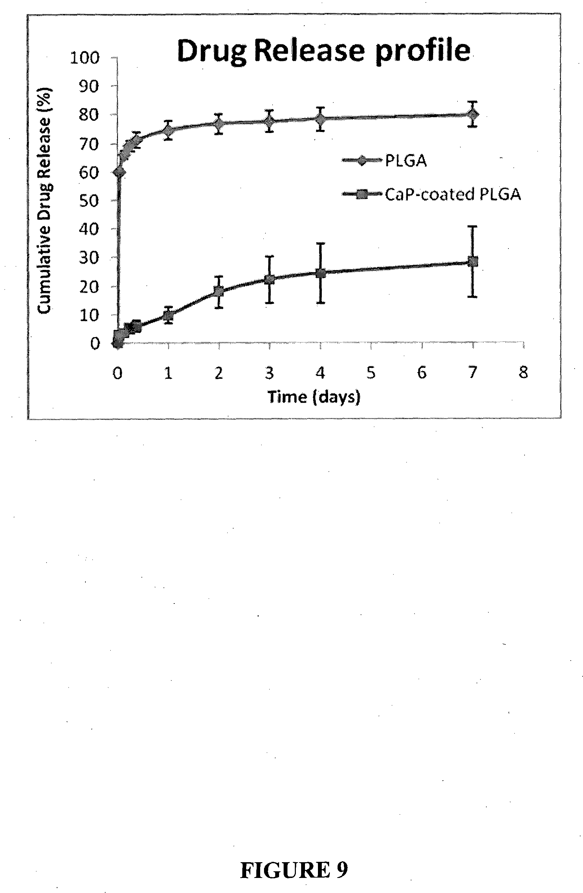

Drug release study of uncoated and coated PLGA particles had also been done in phosphate buffer saline (pH 7.4) at 37°C up to 7 days. The drug used in this study is nafcillin, which is an antibiotic used in the current treatment of osteomyelitis. It is shown from **Figure 9** that the presence of CaP coating on the surface of the particles could reduce burst release drastically. PLGA particles without any coating showed a burst release up to 75% of the total drug encapsulated after 1 day. On the other hand, the ones with CaP coating showed only 10% of the total drug content was released after same period of time. These results show that having CaP coating help overcome burst release of drugs.

By "comprising" it is meant including, but not limited to, whatever follows the word "comprising". Thus, use of the term "comprising" indicates that the listed elements are required or mandatory, but that other elements are optional and may or may not be present.

By "consisting of" is meant including, and limited to, whatever follows the phrase "consisting of". Thus, the phrase "consisting of" indicates that the listed elements are required or mandatory, and that no other elements may be present.

By "about" in relation to a given numberical value, such as for temperature and period of time, it is meant to include numerical values within 10% of the specified value.

## Claims

1. A method for coating particles with calcium phosphate (CaP), wherein the particles are negatively charged, the method comprising:
contacting the particles with a first solution containing calcium ions;
removing the first solution to obtain a precipitate; and
contacting the precipitate with a second solution containing phosphate ions to obtain CaP-coated particles, wherein the negatively charged particles comprise a polymer or polymer mixture, and wherein the second solution is selected from the group consisting of ammonium dihydrogenphosphate solution, disodium hydrogen phosphate, dipotassium hydrogen phosphate, and orthophosphoric acid.

2. The method of claim 1, wherein the polymer is selected from the group consisting of poly(lactic-co-glycolic acid) (PLGA), poly(lactic acid) (PLA), poly(ethyleneglycol) (PEG), poly(L-lactic) (PLLA), polycaprolactone (PCL), and mixtures thereof.

3. The method of claim 1 or 2, wherein the negatively charged particles comprise a negatively charged compound.

4. The method of claim 3, wherein the negatively charged compound is a surfactant.

5. The method of claim 4, wherein the surfactant is selected from the group consisting of sodium dodecyl sulfate (SDS) and poly(ethylene-alt-maleic anhydride) (PEMA).

6. The method of any one of the preceding claims, wherein:
(1) the particles are produced by the emulsion solvent evaporation method comprising dissolving the particle material and a suitable solvent, adding the solution to an aqueous solution containing a negatively charged surfactant to form an emulsion, adding the emulsion to an aqueous solution containing a negatively charged surfactant and evaporating the solvent; and collecting the negatively charged particles from the aqueous solution;
(2) the first solution is selected from the group consisting of calcium nitrate tetrahydrate solution and calcium chloride;
(3) the method further comprises adding a base to the first solution and/or second solution to maintain the pH in the range of 10 to 12;
(4) contacting the particles with the first solution comprises adding the particles to the first solution and stirring the solution for between 5 minutes and 5 hours;
(5) contacting the particles with the second solution comprises adding the precipitate to the second solution and stirring the solution for between 2 minutes and 3 days;
(6) the ratio of concentration of the first solution to the concentration of the second solution is in the range of 5:1 to 1:1; and/or
(7) the particles comprise particles formed of a surface-modified structure with the use of surfactants.

7. The method of any one of the preceding claims, wherein the particles comprise particles comprising a pharmaceutically active compound.

8. The method of claim 7, wherein the pharmaceutically active compound is encapsulated within the polymer.

9. The method of any one of the preceding claims, further comprising separating the CaP-coated particles from the second solution.

10. The method of claim 9, wherein the separation is achieved by centrifuging the mixture to obtain the CaP-coated particles.

11. A nanoparticle obtainable by a method according to any one of claims 1 to 10, comprising a negatively charged particle coated with calcium phosphate (CaP), wherein calcium ions of the CaP are absorbed onto the surface of the negatively charged particle, and wherein the nanoparticle has a diameter of 50 to 500nm.

12. The nanoparticle of claim 11 for use in drug delivery.

13. The nanoparticle for use according to claim 12 for drug delivery to an osseous structure.

## Patentansprüche

1. Ein Verfahren zum Beschichten von Teilchen mit Calciumphosphat (CaP), wobei die Teilchen negativ geladen sind, wobei das Verfahren umfasst:
In-Kontakt-bringen der Teilchen mit einer ersten Lösung, enthaltend Calciumionen;
Entfernen der ersten Lösung, um ein Präzipitat zu erhalten; und
In-Kontakt-bringen des Präzipitats mit einer zweiten Lösung, enthaltend Phosphationen, um CaPbeschichtete Teilchen zu erhalten, wobei die negativ geladenen Teilchen ein Polymer oder eine Polymermischung umfassen, und wobei die zweite Lösung ausgewählt ist aus der Gruppe bestehend aus Ammoniumdihydrogenphosphatlösung, Dinatriumhydrogenphosphat, Dikaliumhydrogenphosphat und Orthophosphorsäure.

2. Das Verfahren gemäß Anspruch 1, wobei das Polymer ausgewählt ist aus der Gruppe bestehend aus Poly(Milchsäure-co-glykolsäure) (PLGA), Polymilchsäure (PLA), Poly(ethylenglykol) (PEG), Poly(L-Milchsäure) (PLLA), Polycaprolacton (PCL) und Mischungen davon.

3. Das Verfahren gemäß Anspruch 1 oder 2, wobei die negativ geladenen Teilchen eine negativ geladene Verbindung umfassen.

4. Das Verfahren gemäß Anspruch 3, wobei die negativ geladene Verbindung ein Tensid ist.

5. Das Verfahren gemäß Anspruch 4, wobei das Tensid ausgewählt ist aus der Gruppe bestehend aus Natriumdodecylsulfat (SDS) und Poly(ethylen-alt-maleinsäureanhydrid) (PEMA).

6. Das Verfahren gemäß einem der vorangegangenen Ansprüche, wobei:
(1) die Teilchen durch das Emulsionslösungsmittelevaporationsverfahren hergestellt werden, umfassend das Lösen des Teilchenmaterials und eines geeigneten Lösungsmittels, Hinzufügen der Lösung zu einer wässrigen Lösung, die ein negativ geladenes Tensid enthält, um eine Emulsion zu bilden, Hinzufügen der Emulsion zu einer wässrigen Lösung, die ein negativ geladenes Tensid enthält und Verdampfen des Lösungsmittels; und Sammeln der negativ geladenen Teilchen aus der wässrigen Lösung;
(2) die erste Lösung ausgewählt ist aus der Gruppe bestehend aus Calciumnitrat-Tetrahydratlösung und Calciumchlorid;
(3) das Verfahren ferner das Hinzufügen einer Base zu der ersten Lösung und/oder zweiten Lösung umfasst, um den pH-Wert im Bereich von 10 bis 12 zu halten;
(4) In-Kontakt-bringen der Teilchen mit der ersten Lösung, das Hinzufügen der Teilchen zu der ersten Lösung und das Rühren der Lösung zwischen 5 Minuten und 5 Stunden umfasst;
(5) In-Kontakt-bringen der Teilchen mit der zweiten Lösung, das Hinzufügen des Präzipitats zu der zweiten Lösung und das Rühren der Lösung zwischen 2 Minuten und 3 Tagen umfasst;
(6) das Verhältnis der Konzentration der ersten Lösung zu der Konzentration der zweiten Lösung in dem Bereich von 5:1 bis 1:1 liegt; und/oder
(7) die Teilchen Teilchen umfassen, die aus einer Oberflächen-modifizierten Struktur unter Verwendung von Tensiden gebildet werden.

7. Das Verfahren gemäß einem der vorangegangenen Ansprüche, wobei die Teilchen Teilchen umfassen, die eine pharmazeutisch aktive Verbindung umfassen.

8. Das Verfahren gemäß Anspruch 7, wobei die pharmazeutisch aktive Verbindung innerhalb des Polymers eingekapselt ist.

9. Das Verfahren gemäß einem der vorangegangenen Ansprüche, ferner umfassend das Trennen der CaP-beschichteten Teilchen von der zweiten Lösung.

10. Das Verfahren gemäß Anspruch 9, wobei die Trennung durch Zentrifugieren der Mischung erreicht wird, um die CaP-beschichteten Teilchen zu erhalten.

11. Ein Nanoteilchen, erhältlich durch ein Verfahren gemäß einem der Ansprüche 1 bis 10, umfassend ein negativ geladenes, mit Calciumphosphat (CaP) beschichtetes Teilchen, wobei Calciumionen des CaP auf der Oberfläche des negativ geladenen Teilchens absorbiert werden und wobei das Nanoteilchen einen Durchmesser von 50 bis 500 nm aufweist.

12. Das Nanoteilchen gemäß Anspruch 11 zur Verwendung in der Wirkstoffabgabe.

13. Das Nanoteilchen zur Verwendung gemäß Anspruch 12 zur Wirkstoffabgabe an eine Knochenstruktur.

## Revendications

1. Procédé pour revêtir des particules avec du phosphate de calcium (CaP), dans lequel les particules sont chargées négativement, le procédé comprenant :
la mise en contact des particules avec une première solution contenant des ions calcium ;
le retrait de la première solution pour que soit obtenu un précipité ; et
la mise en contact du précipité avec une deuxième solution contenant des ions phosphate pour que soient obtenues des particules revêtues de CaP,
dans lequel les particules chargées négativement comprennent un polymère ou un mélange de polymères, et dans lequel la deuxième solution est choisie dans le groupe constitué par une solution de dihydrogénophosphate d'ammonium, l'hydrogénophosphate disodique, l'hydrogénophosphate dipotassique, et l'acide orthophosphorique.

2. Procédé selon la revendication 1, dans lequel le polymère est choisi dans le groupe constitué par le poly(acide lactique-co-glycolique) (PLGA), le poly(acide lactique) (PLA), le polyéthylèneglycol (PEG), le poly(acide L-lactique) (PLLA), la polycaprolactone (PCL), et leurs mélanges.

3. Procédé selon la revendication 1 ou 2, dans lequel les particules chargées négativement comprennent un composé chargé négativement.

4. Procédé selon la revendication 3, dans lequel le composé chargé négativement est un tensioactif.

5. Procédé selon la revendication 4, dans lequel le tensioactif est choisi dans le groupe constitué par le dodécylsulfate de sodium (SDS) et le poly(éthylène-alt-anhydride maléique) (PEMA).

6. Procédé selon l'une quelconque des revendications précédentes, dans lequel :
(1) les particules sont produites par le procédé d'évaporation de solvant en émulsion, comprenant la dissolution du matériau particulaire et d'un solvant convenable, l'ajout de la solution à une solution aqueuse contenant un tensioactif chargé négativement pour former une émulsion, l'ajout de l'émulsion à une solution aqueuse contenant un tensioactif chargé négativement, et l'évaporation du solvant ; et la collecte des particules chargées négativement à partir de la solution aqueuse ;
(2) la première solution est choisie dans le groupe constitué par une solution de nitrate de calcium tétrahydraté et le chlorure de calcium ;
(3) le procédé comprend en outre l'ajout d'une base à la première solution et/ou à la deuxième solution pour maintenir le pH dans la plage allant de 10 à 12 ;
(4) la mise en contact des particules avec la première solution comprend l'ajout des particules à la première solution et l'agitation de la solution pendant une durée comprise entre 5 minutes et 5 heures ;
(5) la mise en contact des particules avec la deuxième solution comprend l'ajout du précipité à la deuxième solution et l'agitation de la solution pendant une durée comprise entre 2 minutes et 3 jours ;
(6) le rapport de la concentration de la première solution à la concentration de la deuxième solution est situé dans la plage allant de 5/1 à 1/1 ; et/ou
(7) les particules comprennent des particules formées d'une structure modifiée en surface par utilisation de tensioactifs.

7. Procédé selon l'une quelconque des revendications précédentes, dans lequel les particules comprennent des particules comprenant un composé pharmaceutiquement actif.

8. Procédé selon la revendication 7, dans lequel le composé pharmaceutiquement actif est encapsulé à l'intérieur du polymère.

9. Procédé selon l'une quelconque des revendications précédentes, comprenant en outre la séparation des particules revêtues de CaP et de la deuxième solution.

10. Procédé selon la revendication 9, dans lequel la séparation est réalisée par centrifugation du mélange pour que soient obtenues les particules revêtues de CaP.

11. Nanoparticule pouvant être obtenue par le procédé selon l'une quelconque des revendications 1 à 10, comprenant une particule chargée négativement revêtue avec du phosphate de calcium (CaP), dans laquelle les ions calcium du CaP sont absorbés sur la surface de la particule chargée négativement, et laquelle nanoparticule a un diamètre compris entre 50 et 500 nm.

12. Nanoparticule selon la revendication 11, pour une utilisation dans la délivrance d'un médicament.

13. Nanoparticule pour une utilisation selon la revendication 12, pour la délivrance d'un médicament à une structure osseuse.
